# NOUVEAU FASCICULE DE BREVET EUROPEEN

(11) **EP 0 686 615 B2**
(45) Date de publication et mention de la décision concernant l'opposition: **27.06.2007**
(45) Mention de la délivrance du brevet: 05.01.2000
(21) Numéro de dépôt: 95401285.2
(22) Date de dépôt: 01.06.1995
(51) Int. Cl.: C07C 5/09, C07C 7/167, B01J 23/58

(54) **Procédé d'hydrogénation catalytique et catalyseur utilisable dans ce procédé**
Verfahren zur katalytischen Hydrierung und in diesem Verfahren zu verwendender Katalysator
Process for the catalytic hydrogenation and catalyst useable in this process

(30) Priorité: 09.06.1994 FR 9407044; 09.06.1994 FR 9407045
(43) Date de publication de la demande: 13.12.1995
(73) Titulaire: Institut Français du Pétrole, 92502 Rueil-Malmaison (FR)
(72) Inventeur: Nguyen Thanh, Canh, La Celle Saint Cloud (FR); Didillon, Blaise, F-92500 Rueil Malmaison (FR); Sarrazin, Patrick, F-92500 Rueil Malmaison (FR); Cameron, Charles, F-75005 Paris (FR)
(74) Mandataire: Hartmann, Günter

(56) Documents cités:
- EP-A- 0 064 301
- EP-A- 0 124 744
- EP-A- 0 519 436
- DD-A- 131 644
- DE-A1- 3 312 252
- FR-A- 1 177 764
- FR-A- 2 338 076
- US-A- 2 475 155
- US-A- 2 802 889
- US-A- 3 243 387
- US-A- 3 651 167
- US-A- 4 061 598
- US-A- 4 404 124
- Bond et al, Trans. Faraday Soc., 54, 1537-1546 (1958)
- Rossington et al, J. of Catalysis, 7, 365-377 (1967)
- Rienaecker et al, Zeitschrift für anorg. u. allg. Chemie, Bd. 336, 259-269 (1965)
- Rienaecker et al, Zeitschrift für anorg. u. allg. Chemie, Bd. 357, 255-263 (1968)
- Télécopie de Süd-Chemie AG à Yanpet, feuille de données 4/90
- Courrier de Süd-Chemie AG à Saudi Yanbu du 03.05.90
- Courrier de Saudi Yanbu à Süd-Chemie AG du 14.08.90 avex remise à jour des données expérimentales
- Courrier Interne de Süd-Chemie AG du 03.02.93
- Ullmanns Encyclopädie der tech. Chemie, 4. Aufl., Bd. 7, 298-299 (1974)

## Description

L'invention concerne un procédé d'hydrogénation sélective en phase gazeuse d'hydrocarbures acétyléniques de 2 ou 3 atomes de carbone (acétylène ou propyne) en les hydrocarbures éthyléniques correspondants (éthylène ou propylène).

Elle concerne également un catalyseur régénérable utilisable dans ce procédé.

L'éthylène est un monomère utilisé pour la préparation d'un grand nombre de polymères. Il est généralement obtenu par des procédés de pyrolyse ou de vapocraquage d'hydrocarbures. L'éthylène ainsi produit contient de faibles quantités d'acétylène (généralement inférieures à 3 %) qu'il est nécessaire d'éliminer avant utilisation. Les teneurs en acétylène dans l'éthylène généralement tolérées pour son utilisation pour la fabrication de polymères sont généralement inférieures à 10 ppm et le plus souvent inférieures à 5 ppm.

Une des techniques utilisées pour éliminer l'acétylène dans l'éthylène est de l'hydrogéner sélectivement en éthylène en présence d'un catalyseur à base de palladium supporté sur un support réfractaire tel que l'alumine. Le problème généralement rencontré avec les catalyseurs monométalliques (constitués uniquement de palladium supporté sur alumine) est que, lorsque les conditions opératoires sont amenées pour permettre l'élimination totale de l'acétylène, une partie de l'éthylène est aussi convertie en éthane. De plus, ces catalyseurs monométalliques présentent généralement des stabilités relativement faibles du fait de la formation importante d'oligomères qui recouvrent progressivement la surface du catalyseur dans les conditions réactionnelles. Ce dépôt hydrocarboné peut certes être éliminé par des procédés d'oxydation ménagée, mais il est avantageux, dans un procédé industriel, d'avoir une durée de fonctionnement du catalyseur entre deux régénérations la plus importante possible.

Pour améliorer les propriétés des catalyseurs, l'ajout au palladium de promoteurs a depuis longtemps été décrit. Ces ajouts peuvent être par exemple l'argent (brevet US-A-2 802 889), le fer et l'argent (brevet US-A-3 243 387).

Ces promoteurs peuvent également être choisis parmi les métaux alcalins ou alcalino-terreux tels que le lithium (brevet US-A-3 325 556), le potassium (demande EP-A-124744) ou le calcium (brevet US-A-4 329 530).

Que ce soit pour les catalyseurs monométalliques (catalyseurs à base de palladium uniquement) ou les catalyseurs promus (catalyseurs comprenant du palladium et au moins un autre élément), il est connu par l'homme du métier que lorsque le palladium est concentré à la surface des billes dudit catalyseur, ses performances catalytiques sont nettement supérieures à celles d'un catalyseur de formule identique pour lequel le palladium est réparti de façon homogène dans les billes de catalyseur. Par exemple dans le cas de l'utilisation des formules bimétalliques palladiumargent, il a été découvert que lorsque le palladium était situé à la périphérie des billes du catalyseur et que l'argent y était réparti de façon homogène, ceci conférait audit catalyseur de meilleures propriétés (Brevets US-A-4 404 124; EP-0064301 et FR-A-2597113), notamment la formation moins importante d'éthane et de produits d'oligomérisation.

On connaît par ailleurs la demande de brevet japonaise JP-A-04 108540 qui décrit des catalyseurs d'hydrogénation sélective en phase liquide du butadiène 1,3, dans lesquels de l'argent est précipité et supporté à la surface du palladium. Dans ces catalyseurs, le support consiste en de l'alumine de surface spécifique relativement élevée et le rapport pondéral Ag/Pd est de 0,3 à 5,0 de préférence de 0,5 à 3,0.

Le document US-A- 3 651 167 décrit un procédé d'hydrogénation sélective en phase gazeuse d'hydrocarbures acétyléniques d'une coupe d'hydrocarbures en C4 pour transformer les composés acétyléniques présents en les composés éthyléniques correspondants sans affecter substantiellement le butadiène. Ce procédé utilise un catalyseur contenant un métal noble du groupe VIII qui peut être réparti, par imprégnation de surface, à la périphérie des particules de catalyseur. Même si ce catalyseur peut contenir en outre un élément du groupe IB, il n'est pas indiqué que cet élément est réparti à la périphérie des particules du catalyseur.

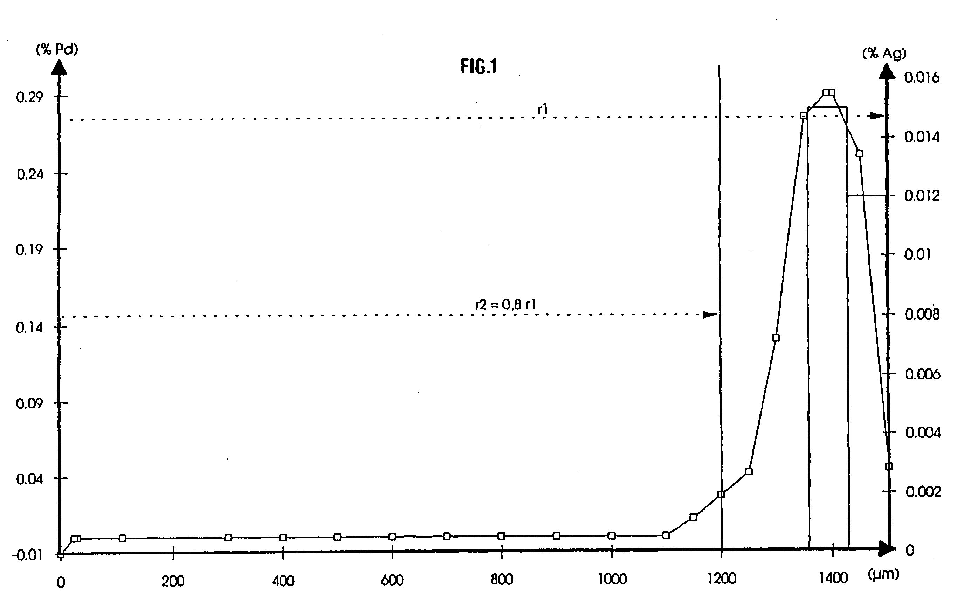

A noter en outre que sont connus, par exemple du document US-A- 3 259 589, des catalyseurs pour le traitement de déchets combustibles dangereux pour les transformer en produits inoffensifs, lesdits catalyseurs contenant, déposé sur un support d'oxyde inorganique réfractaire, au moins un élément métallique actif qui peut être choisi notamment parmi les métaux du groupe du platine, le cuivre, l'argent et l'or. Ce document montre, notamment dans le cas du platine, un mode d'imprégnation de billes d'alumine dans lequel le platine est introduit essentiellement dans une couche continue située à la périphérie des billes (Figure 1), mais il ne décrit pas l'imprégnation de billes de support dans lesquelles deux métaux seraient introduits essentiellement dans une couche localisée à la périphérie de celles-ci.

On a maintenant découvert de façon surprenante qu'il était possible de réaliser avantageusement l'hydrogénation sélective en phase gazeuse des hydrocarbures acétyléniques de 2 ou 3 atomes de carbones (acétylène ou propyne) en les hydrocarbures éthyléniques correspondants (éthylène ou propylène) en utilisant un catalyseur sous forme de billes ou d'extrudés contenant du palladium, au moins un métal du groupe IB de la classification périodique et de l'alumine, dans lequel une proportion d'au moins 80 % du palladium et une proportion d'au moins 80 % du métal du groupe IB sont présentes dans un volume à la périphérie du catalyseur délimité par une surface sphérique ou cylindrique de rayon r₁ correspondant au rayon moyen des billes ou des extrudés de catalyseur et une surface sphérique ou cylindrique de rayon r₂ au moins égal à 0,8 r₁. Le rapport pondéral argent/palladium est compris entre 0,05 et 0,4, de préférence entre 0,05 et 0,25.

Dans le cas de catalyseurs sous forme de billes ou d'extrudés, r₁ et r₂ peuvent être représentés comme suit :

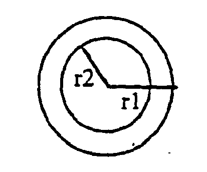

La teneur en palladium est comprise entre 0,01 et 0,5 % en poids du catalyseur. L'élément du groupe IB est le plus souvent l'argent, sous une teneur comprise entre 0,001 et 0,02 % en poids.

Le support utilisé est une alumine alpha. D'une façon courante, il est utilisé sous la forme de billes de diamètres généralement compris entre 2 et 4 mm. Les caractéristiques de l'alumine utilisée sont généralement les suivantes: une surface spécifique comprise entre 5 et 150m²/g et de préférence entre 5 et 60 m²/g ; un volume poreux de 0,3 à 0,95 cm³/g et un diamètre de pores supérieur à 100 Å. Ces différentes caractéristiques sont déterminées par les techniques d'analyse connues par l'homme du métier.

Le palladium peut être introduits selon les techniques connues par l'homme du métier permettant d'obtenir une répartition du palladium à la surface des billes de support, qui correspond aux critères décrits plus haut. La bonne répartition du palladium peut être vérifiée par les techniques classiques telles que par exemple la microsonde de Castaing. Le palladium peut par exemple être introduit par des techniques d'imprégnation de solution aqueuse ou organique d'un précurseur de palladium. Ce précurseur peut par exemple être un composé minéral tel que le chlorure de palladium, le nitrate de palladium, le palladium tétrammine dihydroxyde, le chlorure de palladium tétrammine, ou un composé organométallique, tel que par exemple le palladium bis π allyl ou le palladium bis acétylacétonate.

L'élément du groupe IB, en particulier l'argent, est introduit de telle sorte qu'il reste concentré à la périphérie des billes du support. L'analyse de la teneur en argent après abrasion contrôlée des billes de catalyseur permet de s'assurer de la bonne répartition de l'argent dans les billes de catalyseur. Le précurseur généralement utilisé est le nitrate d'argent. L'acétate d'argent, le citrate d'argent, le chlorure d'argent, le carbonate d'argent peuvent par exemple aussi être utilisés.

Le procédé d'hydrogénation sélective en phase gazeuse des hydrocarbures acétyléniques de 2 ou 3 atomes de carbone en les hydrocarbures éthyléniques correspondants, selon l'invention, peut également être réalisé en utilisant un catalyseur tel qu'il a été défini plus haut mais contenant en outre au moins un métal alcalin ou alcalino-terreux.

La teneur en métal alcalin ou alcalino-terreux du catalyseur est avantageusement choisie pour que le rapport atomique métal alcalin ou alcalino-terreux sur palladium soit compris entre 2 et 20 et de préférence entre 4 et 15. De préférence cette teneur est comprise entre 0,05 % et 0,2 % poids du catalyseur.

A titre de métal alcalin, on met en jeu de préférence le sodium ou le potassium.

Le métal alcalin ou alcalino-terreux est introduit selon les techniques connues par l'homme du métier. Les précurseurs généralement utilisés sont les nitrates, les acétates, les chlorures les carbonates, les hydroxydes.

Le palladium et le métal du groupe IB et éventuellement le métal alcalin ou alcalino-terreux peuvent être introduits à partir d'une solution commune de leurs précurseurs ou à partir de solutions séparées contenant chacune un ou deux éléments. Dans ce dernier cas, des traitements de séchage, de calcination ou de réduction à des températures comprises entre 120°C et 900°C peuvent être réalisés entre deux étapes d'imprégnation consécutives.

Lorsque le palladium et l'élément du groupe IB (plus particulièrement l'argent) sont introduits à partir de solutions différentes, les techniques de préparation qui peuvent être employées sont par exemple celles décrites dans le brevet US-A-4 533 779, qui utilise le chlorure d'agent comme précurseur ou dans le brevet US-A-4 504 593, qui utilise le citrate d'argent comme précurseur.

Le catalyseur ainsi obtenu est généralement séché à des températures comprises entre la température ambiante et 150°C. Le catalyseur ainsi séché peut être utilisé tel que ou le plus souvent il est de préférence calciné afin de décomposer les précurseurs métalliques et/ou réduit avant utilisation. La calcination est généralement réalisée en traitant ledit catalyseur sous flux d'air à une température comprise entre 400°C et 900°C. La réduction peut être réalisée par traitement du catalyseur par un gaz contenant de l'hydrogène à une température comprise entre la température ambiante et 500°C.

Le procédé d'hydrogénation de l'invention s'applique plus particulièrement à l'hydrogénation de l'acétylène présent dans un gaz contenant de l'éthylène. Afin de s'approcher des conditions réactionnelles qui permettent d'éliminer totalement l'acétylène, le rapport molaire hydrogène sur acétylène est généralement compris entre 1 et 2, la température de la réaction est généralement comprise entre 25 et 100 °C, la pression est généralement comprise entre 1 et 5 MPa. Le débit de charge exprimé en litre de charge gazeuse par litre de catalyseur et par heure est généralement compris entre 1000 et 10 000 h⁻¹.

Au cours de l'utilisation, le catalyseur se désactive du fait d'un dépôt de composés hydrocarbonés recouvrant progressivement la phase active. Lorsque les performances du catalyseur sont jugées insuffisante, le catalyseur peut être régénéré. La régénération du catalyseur est réalisée par combustion contrôlée des espèces hydrocarbonées présentes sur celui-ci. Cette combustion est réalisée dans les conditions connues de l'homme du métier, généralement en chauffant progressivement le catalyseur en présence d'un gaz contenant de l'oxygène à une température comprise entre 350 et 500°C.

La présente invention concerne également à titre de produits nouveaux, les catalyseurs contenant du palladium et au moins un élément du groupe IB (en particulier l'argent) tels qu'ils ont été définis plus haut et présentant un rapport pondéral du métal du groupe IB (en particulier de l'argent) au palladium de 0,05 à 0,25.

L'invention concerne enfin les catalyseurs ainsi définis, contenant en outre au moins un métal alcalin ou alcalino-terreux.

Les exemples qui suivent illustrent l'invention. Les exemples 3, 4, 10 et 11 sont donnés à titre de comparaison.

### EXEMPLE 1 : Préparation du catalyseur A (selon l'invention)

Un catalyseur selon l'invention (Catalyseur A) est préparé par imprégnation de 100 g d'un support à base d'alumine alpha par 60 ml d'une solution d'acide nitrique, de nitrate de palladium et de nitrate d'argent. Le support utilisé se présente sous forme de billes de 2 à 4 mm de diamètre ayant une surface spécifique de 10 m²/g et un volume poreux de 0,6 cm³/g. Après imprégnation, le catalyseur est séché à 120 °C et calciné sous air à 750 °C. Le catalyseur A ainsi obtenu contient 0,05 % en poids de palladium et 0,005 % en poids d'argent. La répartition moyenne des éléments dans les grains de catalyseurs, est représentée figure 1. Sur le diagramme, on a porté en abscisses les rayons en micromètres et en ordonnées, à gauche, la concentration pondérale locale en palladium et à droite, la concentration pondérale locale en argent représentée sous forme d'histogramme.

Ces analyses montrent que 84 % de l'argent est concentré dans un volume délimité par une sphère de rayon r₁ de 1,5 mm et une sphère de rayon r2 de 1,39 mm. Le rapport r₂/r₁ est donc égal à 0,93 et donc bien supérieur à 0,8. En ce qui concerne le palladium, 94 % du palladium est concentré dans un volume délimité par une sphère de rayon r₁ de 1,5 mm et une sphère de rayon r₂ de 1,2 mm. Le rapport r₂/r₁ est ici égal à 0,8. La répartition des éléments dans le grain de catalyseur est donc bien conforme à l'invention.

### EXEMPLE 2 : Préparation du catalyseur B (selon l'invention)

Un catalyseur selon l'invention (Catalyseur B) est préparé par imprégnation de 100 g d'un support à base d'alumine alpha par 60 ml d'une solution d'acide nitrique, de nitrate de palladium et de nitrate d'argent. Le support utilisé se présente sous forme de billes de 2 à 4 mm de diamètre ayant une surface spécifique de 10 m²/g et un volume poreux de 0,6 cm³/g. Après imprégnation, le catalyseur est séché à 120 °C et calciné sous air à 750 °C. Le catalyseur B ainsi obtenu contient 0,05 % en poids de palladium et 0,010 % en poids d'argent. La répartition moyenne des éléments dans les grains de catalyseurs est conforme à l'invention.

### EXEMPLE 3 : Préparation du catalyseur C (comparatif)

Un catalyseur C est préparé selon la même procédure que dans l'exemple 1 mais en utilisant une solution d'imprégnation contenant de l'acide nitrique et du nitrate de palladium. Le catalyseur C ainsi obtenu contient 0,05 % de palladium. L'analyse par microsonde de Castaing des catalyseurs A et C ne permet pas de mettre en évidence des différences significatives de répartition du palladium entre ces deux échantillons.

### EXEMPLE 4 : Préparation du catalyseur D (comparatif)

Un catalyseur D est préparé en immergeant à température ambiante 100 g de support dans 120 ml d'une solution aqueuse de nitrate d'argent contenant 8 mg d'argent. Le catalyseur est laissé quelques minutes sous agitation. La solution surnageante est alors éliminée. Le catalyseur est alors séché à 120 °C et calciné à 500 °C. Sur ce catalyseur, on imprègne alors 60 ml d'une solution d'acide nitrique et de nitrate de palladium. Après imprégnation le catalyseur est séché à 120 °C et calciné sous air à 750 °C. Le catalyseur D ainsi obtenu contient 0,05 % en poids de palladium et 0,005 % en poids d'argent. L'analyse par microsonde de Castaing des catalyseurs A et D ne permet pas de mettre en évidence des différences significatives de répartition du palladium dans ces deux échantillons. Par contre, l'analyse de la teneur en argent après abrasion contrôlée des billes de catalyseur ne permet pas d'identifier de différence de concentration en argent dans les billes de catalyseur.

### EXEMPLE 5 : Préparation du catalyseur E (selon l'invention)

50 g du catalyseur monométallique C sont réduits en solution aqueuse par l'acide citrique. Dans cette solution, on introduit alors 4 mg d'acétate d'argent. Le système réactionnel est maintenu sous faible agitation pendant 8 heures. Le catalyseur est alors filtré, séché 16 h à 120°C et calciné pendant 2 heures à 750 °C. Le catalyseur A ainsi obtenu contient 0,05 % en poids de palladium et 0,005 % en poids d'argent. La répartition moyenne des éléments dans les grains de catalyseurs E est conforme à l'invention.

### EXEMPLE 6 : Comparaison des propriétés hydrogénantes des différents catalyseurs

Les tests catalytiques sont réalisés sur les catalyseurs A, B, C, D et E pour déterminer leur sélectivité et leur stabilité lors de l'hydrogénation de l'acétylène contenu dans une charge contenant 98 % d'éthylène et 2 % d'acétylène.

15 ml du catalyseur à tester sont d'abord placés dans un réacteur vertical en acier. Ce réacteur est alors placé dans un four permettant de contrôler la température. Dans un premier temps le catalyseur est réduit sous courant d'hydrogène à 150 °C pendant 2 heures sous pression atmosphérique. La température est alors portée à 50 °C, le débit d'hydrogène à 1,5 l.h⁻¹ et la pression à 2,5 MPa. La charge, composée de 98 % d'éthylène et de 2 % d'acétylène est alors injectée avec un débit volumique correspondant à une vitesse spatiale de 3300 h⁻¹. L'analyse de l'effluent gazeux en sortie de réacteur est réalisée par chromatographie en phase gazeuse. Dans ces conditions, la stabilité du catalyseur est définie comme étant le temps à partir duquel de l'acétylène est détecté en sortie de réacteur. La sélectivité du catalyseur correspond à la teneur en éthylène de la charge après élimination totale de l'acétylène. Les résultats obtenus sont rapportés dans le tableau I.

**Tableau 1.**

| ***Comparaison des performances des catalyseurs A, B, C, D et E pour l'hydrogénation de l'acétylène.*** | | |
|---|---|---|
| Catalyseurs | Stabilité des catalyseurs (heures) | Sélectivité des catalyseurs (%) |
| Catalyseur A (selon l'invention) | 96 | 98,5 |
| Catalyseur B (selon l'invention) | 85 | 98,5 |
| Catalyseur C (comparatif) | 22 | 98,3 |
| Catalyseur D (comparatif) | 36 | 98,2 |
| Catalyseur E (selon l'invention) | 94 | 98,5 |

Ces résultats montrent clairement que les catalyseurs selon l'invention (catalyseurs A, B ou E) présentent des performances catalytiques (stabilité et sélectivité) supérieures à celles des catalyseurs monométalliques (catalyseur C) ou celles de catalyseurs où l'argent est réparti uniformément dans les billes du catalyseur (catalyseur D).

### EXEMPLE 7 : Régénération d'un catalyseur selon l'invention

Après utilisation du catalyseur A pendant 120 heures dans les conditions de l'exemple 6, le catalyseur A est régénéré. Dans cette procédure de régénération, le catalyseur est porté à 200 °C sous azote, puis traité sous air dilué à une température comprise entre 200 et 500 °C pour brûler les composés hydrocarbonés présents sur le catalyseur.

Après régénération, les performances du catalyseur A régénéré sont évaluées dans les conditions de l'exemple 6. Les performances d'un tel système régénéré sont rapportées dans le tableau 2.

**Tableau 2**

| Catalyseurs | Stabilité des catalyseurs (heures) | Sélectivité des catalyseurs (%) |
|---|---|---|
| Catalyseur A (selon l'invention) | 96 | 98,5 |
| Catalyseur A Régénéré | 95 | 98,7 |

Ces résultats montrent que, aux erreurs expérimentales près, le catalyseur A régénéré présente les mêmes performances en hydrogénation de l'acétylène que le catalyseur neuf.

### EXEMPLE 8 : Préparation du catalyseur F (selon l'invention)

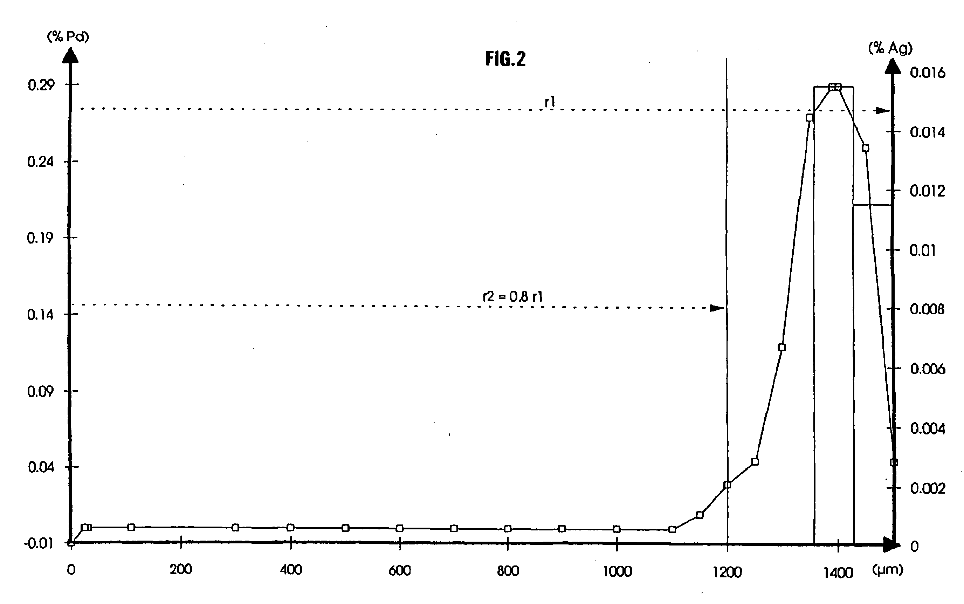

Un catalyseur selon l'invention (Catalyseur F) est préparé par imprégnation de 100 g d'un support à base d'alumine alpha par 60 ml d'une solution d'acide nitrique, de nitrate de palladium, de nitrate d'argent et de nitrate de sodium. Le support utilisé se présente sous forme de billes de 2 à 4 mm de diamètre ayant une surface spécifique de 10 m²/g et un volume poreux de 0,6 cm³/g. Après imprégnation, le catalyseur est séché à 120 °C et calciné sous air à 750 °C. Le catalyseur F ainsi obtenu contient en poids 0,05 % de palladium, 0,005% d'argent et 0,05 % de sodium. La répartition moyenne des éléments métalliques dans les grains de catalyseur est représentée figure 2.

Sur le diagramme on a porté, en abscisses, les rayons en micromètres et, en ordonnées, à gauche les concentrations pondérales locales en palladium réprésentées par des (□) et à droite, la concentration pondérale locale en argent, sous forme d'histogramme.

Ces analyses montrent que 84% de l'argent est concentré dans un volume délimité par une sphère de rayon r₁ de 1,5 mm et une sphère de rayon r₂ de 1,39 mm. Le rapport r₂/r₁ est donc égal à 0,93 et donc supérieur à 0,8. En ce qui concerne le palladium, 94% du palladium est concentré dans un volume délimité par une sphère de rayon r₁ de 1,5 mm et une sphère de rayon r₂ de 1,2 mm. Le rapport r₂/r₁ est ici égal à 0,8. La répartition des éléments dans le grain de catalyseur est donc bien conforme à l'invention.

### EXEMPLE 9 : Préparation du catalyseur G (selon l'invention)

Un catalyseur selon l'invention (Catalyseur G) est préparé par imprégnation de 100 g d'un support à base d'alumine alpha par 60 ml d'une solution d'acide nitrique, de nitrate de palladium, de nitrate d'argent et de nitrate de sodium. Le support utilisé se présente sous forme de billes de 2 à 4 mm de diamètre ayant une surface spécifique de 10m²/g et un volume poreux de 0,6 cm³/g. Après imprégnation, le catalyseur est séché à 120°C et calciné sous air à 750°C. Le catalyseur G ainsi obtenu contient en poids 0,05% de palladium, 0,010% d'argent et 0,05% de sodium. La répartition moyenne des éléments dans les grains de catalyseurs est conforme à l'invention.

### EXEMPLE 10 : Préparation du catalyseur H (comparatif)

Un catalyseur H est préparé selon la même procédure que dans l'exemple 8, mais en utilisant une solution d'imprégnation contenant de l'acide nitrique, du nitrate de palladium et du nitrate de sodium.. Le catalyseur H ainsi obtenu contient en poids 0,05% de palladium et 0,05 % de sodium. L'analyse par microsonde de Castaing des catalyseurs F et H ne permet pas de mettre en évidence des différences significatives de répartition du palladium entre ces deux échantillons.

### EXEMPLE 11 : Préparation du catalyseur I (Comparatif)

Un catalyseur I est préparé en immergeant à température ambiante 100 g de support dans 120 ml d'une solution aqueuse de nitrate d'argent contenant 8 mg d'argent. Le catalyseur est laissé quelques minutes sous agitation. La solution sunageante est alors éliminée. Le catalyseur est alors séché à 120 °C et calciné à 500 °C. Sur ce catalyseur, on imprègne alors 60 ml d'une solution d'acide nitrique, de nitrate de palladium et nitrate de sodium. Après imprégnation, le catalyseur I ainsi obtenu contient en poids 0,05% de palladium, 0,005% d'argent et 0,05% de sodium. L'analyse par microsonde de Castaing des catalyseurs F et I ne permet pas de mettre en évidence des différences significatives de répartition du palladium dans ces deux échantillons. Par ailleurs, l'analyse de la teneur en argent après abrasion contrôlée des billes de catalyseur, ne permet pas d'identifier de différence de concentration en argent dans les billes de catalyseur.

### EXEMPLE 12 : Préparation du catalyseur J (selon l'invention)

Un catalyseur selon l'invention (Catalyseur J) est préparé par imprégnation de 100 g d'un support à base d'alumine alpha par 60 ml d'une solution d'acide nitrique, de nitrate de palladium, de nitrate d'argent et de nitrate de sodium. Le support utilisé se présente sous forme de billes de 2 à 4 mm de diamètre ayant une surface spécifique de 10m²/g et un volume poreux de 0,6 cm³/g. Après imprégnation, le catalyseur est séché à 120°C et calciné sous air à 750°C. Le catalyseur J ainsi obtenu contient en poids 0,05% de palladium, 0,020% d'argent et 0,05% de sodium. La répartition moyenne des éléments dans les grains de catalyseurs est conforme à l'invention.

### EXEMPLE 13 : Préparation du catalyseur K (selon l'invention)

Un catalyseur selon l'invention (Catalyseur K) est préparé par imprégnation de 100 g d'un support à base d'alumine alpha par 60 ml d'une solution d'acide nitrique, de nitrate de palladium, de nitrate d'argent et de nitrate de sodium. Le support utilisé se présente sous forme de billes de 2 à 4 mm de diamètre ayant une surface spécifique de 10m²/g et un volume poreux de 0,6 cm³/g. Après imprégnation le catalyseur est séché à 120°C et calciné sous air à 750°C. Le catalyseur K ainsi obtenu contient en poids 0,05% de palladium, 0,010% d'argent et 0,1% de sodium. La répartition moyenne des éléments dans les grains de catalyseurs est conforme à l'invention.

### EXEMPLE 14 : Comparaison des propriétés hydrogénantes des différents catalyseurs

Les tests catalytiques sont réalisés sur les catalyseurs F, G, H, I, J et K pour déterminer leur sélectivité et leur stabilité lors de l'hydrogénation de l'acétylène contenu dans une charge contenant 98 % en poids d'éthylène et 2 % en poids d'acétylène.

15 ml du catalyseur à tester sont d'abord placés dans un réacteur vertical en acier. Ce réacteur est alors placé dans un four permettant de contrôler la température. Dans un premier temps le catalyseur est réduit sous courant d'hydrogène à 150°C pendant 2 heures sous pression atmosphérique. La température est alors portée à 50°C, le débit d'hydrogène à 1,5 l.h⁻¹ et la pression à 2,5 MPa. La charge, composée de 98 % en poids d'éthylène et de 2 % en poids d'acétylène est alors injectée avec un débit volumique correspondant à une vitesse spatiale de 3300 h⁻¹. L'analyse de l'effluent gazeux en sortie de réacteur est réalisée par chromatographie en phase gazeuse. Dans ces conditions, la stabilité du catalyseur est définie comme étant le temps à partir duquel de l'acétylène est détecté en sortie de réacteur. La sélectivité du catalyseur correspond à la teneur en éthylène de la charge après élimination totale de l'acétylène. Les résultats obtenus sont rapportés dans le tableau 3.

**Tableau 3.**

| ***Comparaison des performances des catalyseurs F, G, H, I, J et K pour l'hydrogénation de l'acétylène.*** | | |
|---|---|---|
| Catalyseurs | Stabilité des catalyseurs (heures) | Sélectivité des catalyseurs (%) |
| Catalyseur F (selon l'invention) | 105 | 98,2 |
| Catalyseur G (selon l'invention) | 120 | 98,3 |
| Catalyseur H (comparatif) | 65 | 98,3 |
| Catalyseur I (comparatif) | 66 | 98,2 |
| Catalyseur J (selon l'invention) | 101 | 98,3 |
| Catalyseur K (selon l'invention) | 121 | 98,7 |

Ces résultats montrent clairement que les catlyseurs selon l'invention (catalyseurs F, G, J ou K) présentent des performances catalytiques (stabilité et sélectivité) supérieures à celles de catalyseurs monométalliques (catalyseur H) ou celles de catalyseurs où l'argent est réparti uniformément dans les billes de catalyseur (catalyseur I).

### EXEMPLE 15 : Régénération d'un catalyseur selon l'invention

Après utilisation du catalyseur F pendant 120 heures dans les conditions de l'exemple 14, le catalyseur F est régénéré. Dans cette procédure de régénération le catalyseur est porté à 200 °C sous azote, puis traité sous air dilué à une température comprise entre 200 et 500 °C pour brûler les composés hydrocarbonés présents sur le catalyseur.

Après régénération, les performances du catalyseur F régénéré sont évaluées dans les conditions de l'exemple 14. Les performances d'un tel système régénéré sont rapportées dans le tableau 4 suivant:

**Tableau 4:**

| Catalyseurs | Stabilité des catalyseurs (heures) | Sélectivité des catalyseurs (%) |
|---|---|---|
| Catalyseur F (selon l'invention) | 105 | 98,5 |
| Catalyseur F Régénéré | 104 | 98,7 |

Ces résultats montrent, qu'aux erreurs expérimentales près, le catalyseur F régénéré présente les mêmes performances en hydrogénation de l'acétylène que le catalyseur neuf.

## Revendications

1. Procédé d'hydrogénation sélective en phase gazeuse d'au moins un hydrocarbure acétylénique de 2 ou 3 atomes de carbone en l'hydrocarbure éthylénique correspondant, comprenant le passage en phase gazeuse d'une charge comprenant au moins un hydrocarbure acétylénique de 2 ou 3 atomes de carbone en présence d'hydrogène sur un catalyseur sous forme de billes ou d'extrudés cylindriques comprenant du palladium, au moins un métal du groupe IB de la classification périodique et de l'alumine, ledit procédé étant
**caractérisé**
**en ce que** dans ledit catalyseur, le rapport pondéral du métal du groupe IB au palladium est de 0,05 à 0,4,
**en ce qu'**une proportion d'au moins 80 % du palladium et une proportion d'au moins 80 % du métal du groupe IB sonst présentes dans un volume à la périphérie du catalyseur délimité par une surface sphérique ou cylindrique de rayon r₁ correspondant au rayon moyen des billes ou des extrudés de catalyseur et une surface sphérique ou cylindrique.de rayon r₂ au moins égal à
0, 8 r₁ et en ce que
• ladite alumine est une alumine alpha;
• la teneur en palladium est de 0,01 à 0,5 % en poids; et
• la teneur en métal du groupe IB est de 0,001 à 0,02 % en poids.

2. Procédé selon la revendication 1, **caractérisé en ce que** l'alumine a une surface spécifique de 5 à 60 m²/g;

3. Procédé selon la revendication 1 ou 2, **caractérisé en ce que** le métal du groupe IB est l'argent.

4. Procédé selon l'une des revendications 1 à 3, **caractérisé en ce que** le rapport pondéral du métal du groupe IB au palladium est de 0.05 à 0,25.

5. Procédé selon l'une des revendications 1 à 4, **caractérisé en ce que** le catalyseur comprend en outre au moins un métal alcalin ou alcalino-terreux.

6. Procédé selon l'une des revendications 1 à 5, **caractérisé en ce que**, dans le catalyseur, le rapport atomique métal alcalin ou alcalino-terreux sur palladium et de 2 à 20.

7. Procédé selon l'une des revendications 5 et 6, **caractérisé en ce que**, la tenueur du catalyseur en métal alcalin ou alcalino-terreux est de 0,05 à 0.2% en poids.

8. Catalyseur sous forme de billes ou d'extrudés comprenant du palladium, au moins un métal du groupe IB de la classification périodique et de l'alumine, **caractérisé**
**en ce que** le rapport pondéral du métal du grope IB au palladium est de 0,05 à 0,25,
**en ce qu'**une proportion d'au moins 80 % du palladium et une proportion d'au moins 80 % du métal du groupe IB sont présentes dans un volume à la périphérie du catalyseur délimité par une surface sphérique ou cylindrique de rayon r₁ correspondant au rayon moyen des billes ou des extrudés de catalyseur et une surface sphérique ou cylindrique de rayon r₂ au moins égal à 0,8 r₁ et en ce que
• ladite alumine est une alumine alpha;
• sa teneur en palladium est de 0,01 à 0,5 % en poids; et
• sa teneur en métal du groupe IB est de 0,001 à 0,02 % en poids.

9. Catalyseur selon la revendication 8, **caractérisé en ce que** l'alumine a une surface spécifique de 5 à 60 m²/g.

10. Catalyseur selon la revendication 8 ou 9, **caractérisé en ce que** le métal du groupe IB est l'argent.

11. Catalyseur selon l'une des revendications 8 à 10, **caractérisé en ce qu'**il comprend en outre au moins un métal alcalin ou aicatino-terreux.

12. Catalyseur selon l'une des revendications 8 à 11, **caractérisé en ce** le rapport atomique métal alcalin ou alcalino-terreux sur palladium est de 2 à 20.

13. Catalyseur selon la revendication 11 ou 12, **caractérisé en ce** sa teneur en métal alcalin ou aicalino-terreux est de 0,05 à 0.2% en poids.

## Claims

1. A process of selective gas-phase hydrogenation of at least one acetylenic hydrocarbon with 2 or 3 carbon atoms in the corresponding ethylenic hydrocarbon, comprising the passing of a charge of at least one acetylenic hydrocarbon with 2 or 3 carbon atoms in the presence of hydrogen over a catalyst in the form of balls or cylindrical pellets ("extrudés") and comprising palladium, at least one metal from Group IB of the Periodic Table and alumina, **characterized in that** in the aforesaid catalyst the weight ratio of the Group IB metal to palladium is 0.05 to 0.4, **in that** amounts of at least 80 % of the palladium and of at least 80 % of the Group IB metal are present within a peripheral catalyst volume defined by a spherical or cylindrical surface having a radius r₁ equal to the average radius of said catalyst balls or pellets, respectively, and by a spherical or cylindrical surface having a radius r₂ equal to at least 0,8•r₁, and **in that**
• the alumina comprises alpha-alumina,
• the palladium comprises 0.01 to 0.5 wt.% and
• the Group IB metal comprises 0.001 to 0.02 wt.%.

2. Process as in claim 1, **characterized in that** the alumina has a specific surface area of 5 to 60 sq.m. per gram.

3. Process as in claim 1 or 2, **characterized in that** the Group IB metal is silver.

4. Process as in any of claims 1 to 3, **characterized in that** the weight ratio of the Group IB metal to palladium is 0.05 to 0.25.

5. Process as in any of claims 1 to 4, **characterized in that** the catalyst further comprises at least one alkali or earth alkali metal.

6. Process as in any of claims 1 to 5, **characterized in that**, in said catalyst, the atom ratio of said alkali or earth alkali metal to palladium is 2 to 20.

7. Process as in claim 5 or 6, **characterized in that** the alkali or earth alkali metal in the catalyst amounts to 0.05 to 0.2 wt.%.

8. A catalyst in the form of balls or pellets and comprising palladium, at least one metal from Group IB of the Periodic Table and alumina, **characterized in that** the weight ratio of the Group IB metal to palladium is 0.05 to 0.25, **in that** at least 80 % of the palladium and at least 80 % of the Group IB metal are present within a peripheral catalyst volume which is defined by a spherical or cylindrical surface having a radius r₁ corresponding to the average radius of the catalyst spheres or pellets, respectively, and by a spherical or cylindrical surface having a radius r₂ equal to at least 0.8•r₁, and **in that**
• the alumina comprises alpha-alumina;
• the palladium amounts to 0.01 to 0.5 wt. % and
• the Group IB metal amounts to 0.001 to 0.02 wt. %.

9. Catalyst as in claim 8, **characterized in that** the alumina has a specific surface area of 5 to s 60 sq.m. per gram.

10. Catalyst as in claim 8 or 9, **characterized in that** the Group IB metal is silver.

11. Catalyst as in any of claims 8 to 11, **characterized in that** the catalyst further comprises at least one alkali or earth alkali metal.

12. Process as in any of claims 8 to 11, **characterized** that the atom ratio of the alkali or earth alkali metal to the palladium in the catalyst is 2 to 20.

13. Process as in claim 11 or 12, **characterized in that** the alkali or earth alkali metal in the catalyst amounts to 0.05 to 0.2 wt.-%.

## Patentansprüche

1. Verfahren zur selektiven Gasphasen-Hydrierung mindestens eines acetylenischen Kohlenwasserstoffs mit 2 oder 3 C-Atomen im entsprechenden ethylenischen Kohlenwasserstoff, bei dem man eine Charge mit mindestens einem acetylischen Kohlenwasserstoff mit 2 oder 3 C-Atomen in Gegenwart von Wasserstoff über einen Katalysator in Form von Kugeln oder zylindrischen Pellets ("extrudés") leitet, der Palladium, mindestens ein Metall der Gruppe IB des Periodischen Systems und Aluminiumoxid ("alumina") aufweist, **dadurch gekennzeichnet, dass** beim genannten Katalysator das Gewichtsverhältnis des Metalls der Gruppe IB zum Palladium 0,05 bis 0,4 beträgt, dass ein Anteil von mindestens 80 % des Palladiums und ein Anteil von mindestens 80 % des Metalls der Gruppe IB in einem Volumen am Umfang des Katalysators vorliegen, das begrenzt ist durch eine Kugel- bzw. Zylinderfläche mit dem Radius r₁ entsprechend dem durchschnittlichen Radius der Kugel oder der Pellets des Katalysators und durch eine Kugel- bzw. Zylinderfläche des Radius r₂ gleich min. 0,8•r₁, dass
• das Aluminiumoxid ein Alpha-Aluminiumoxid ist und dass
• der Palladiumgehalt 0,01 bis 0,5 Gew.-% und
• der Gehalt am Metall der Gruppe IB 0,001 bis 0,02 Gew.-% betragen.

2. Verfahren nach Anspruch 1, **dadurch gekennzeichnet, dass** das Aluminiumoxid eine spezifische Oberfläche von 5 bis 60 m²/g hat.

3. Verfahren nach Anspruch 1 oder 2, **dadurch gekennzeichnet, dass** das Metall der Gruppe IB Silber ist.

4. Verfahren nach einem der Ansprüche 1 bis 3, **dadurch gekennzeichnet, dass** das Gewichtsverhältnis des Metalls der Gruppe IB zum Palladium 0,05 bis 0,25 beträgt.

5. Verfahren nach einem der Ansprüche 1 bis 4, **dadurch gekennzeichnet, dass** der Katalysator weiterhin mindestens ein Alkali- oder Erdalkalimetall aufweist.

6. Verfahren nach einem der Ansprüche 1 bis 5, **dadurch gekennzeichnet, dass** in dem Katalysator das Atomverhältnis Alkali- oder Erdalkalimetall zum Palladium 2 bis 20 beträgt.

7. Verfahren nach Anspruch 5 oder 6, **dadurch gekennzeichnet, dass** der Gehalt an Alkali- oder Erdalkalimetall im Katalysator 0,05 bis 0,2 Gew.-% beträgt.

8. Katalysator in Form von Kugeln oder Pellets, der Palladium, mindestens ein Metall der Gruppe IB des Periodischen Systems und Aluminiumoxid ("alumina") aufweist, **dadurch gekennzeichnet ist, dass** das Gewichtsverhältnis des Metalls der Gruppe IB zum Palladium 0,05 bis 0,25 beträgt, dass ein Anteil von mindestens 80 % des Palladiums und ein Anteil von mindestens 80 % des Metalls der Gruppe IB in einem Volumen am Umfang des Katalysators vorliegen, das begrenzt ist durch eine Kugel- bzw. Zylinderfläche mit dem Radius r₁ entsprechend dem durchschnittlichen Radius der Kugel oder der Pellets des Katalysators und durch eine Kugel- bzw. Zylinderfläche des Radius r₂ gleich min. 0,8•r₁, dass
• das Aluminiumoxid ein Alpha-Aluminiumoxid ist und dass
• der Palladiumgehalt 0,01 bis 0,5 Gew.-% und
• der Gehalt am Metall der Gruppe IB 0,001 bis 0,02 Gew.-% betragen.

9. Katalysator nach Anspruch 8, **dadurch gekennzeichnet, dass** das Aluminiumoxid eine spezifische Oberfläche von 5 bis 60 m²/g hat.

10. Verfahren nach Anspruch 8 oder 9, **dadurch gekennzeichnet, dass** das Metall der Gruppe IB Silber ist.

11. Verfahren nach einem der Ansprüche 8 bis 11, **dadurch gekennzeichnet, dass** der Katalysator weiterhin mindestens ein Alkali- oder Erdalkalimetall aufweist.

12. Verfahren nach einem der Ansprüche 8 bis 11, **dadurch gekennzeichnet, dass** in dem Katalysator das Atomverhältnis Alkali- oder Erdalkalimetall zum Palladium 2 bis 20 beträgt.

13. Verfahren nach Anspruche 11 oder 12, **dadurch gekennzeichnet, dass** der Gehalt an Alkali- oder Erdalkalimetall im Katalysator 0,05 bis 0,2 Gew.-% beträgt.
